# EUROPEAN PATENT APPLICATION

(11) **EP 4 354 143 A1**
(43) Date of publication of application: **17.04.2024**
(21) Application number: 22820344.4
(22) Date of filing: 10.06.2022
(51) Int. Cl.: G01N 33/574, C07K 16/18, C12Q 1/6844, C12Q 1/6886, G01N 33/53

(54) **KIT FOR DIAGNOSIS OF CANCER AND USE THEREOF**

(30) Priority: 10.06.2021 JP 2021097456; 10.12.2021 JP 2021201094
(71) Applicant: The Kitasato Institute, Tokyo 108-8641 (JP)
(72) Inventor: MATSUMOTO, Kazumasa, Sagamihara-shi, Kanagawa 252-0373 (JP); SATO, Yuichi, Sagamihara-shi, Kanagawa 252-0373 (JP); AMANO, Noriyuki, Sagamihara-shi, Kanagawa 252-0373 (JP); TASHIRO, Yuriko, Sagamihara-shi, Kanagawa 252-0373 (JP); IWAMURA, Masatsugu, Sagamihara-shi, Kanagawa 252-0373 (JP)
(74) Representative: Regimbeau
(86) International application number: PCT/JP2022/023474
(87) International publication number: WO 2022/260166

(57) **Abstract**

A kit for diagnosis of cancer, including a specific binding substance to a SPRY domain-containing SOCS box protein 2 (SPSB2) protein, a primer set for amplifying cDNA of an SPSB2 gene, or a probe that specifically hybridizes to mRNA of an SPSB2 gene, a kit for determining a prognosis of a patient with cancer, a method for determining a biological sample, a method for collecting data for determining whether or not a subject is affected by cancer, and a method for predicting a prognosis of a patient with cancer are provided.

## Description

### [Technical Field]

The present invention relates to a kit for diagnosis of cancer and a use thereof. More specifically, the present invention relates to a kit for diagnosis of cancer, a kit for determining a prognosis of a patient with cancer, a method for determining a biological sample, a method for collecting data for determining whether or not a subject is affected by cancer, a method for predicting a prognosis of a patient with cancer, and a method for screening an anti-cancer agent. Priority is claimed on Japanese Patent Application No. 2021-097456, filed in Japan on June 10, 2021, and Japanese Patent Application No. 2021-201094 filed in Japan on December 10, 2021, the contents of which are incorporated herein by reference.

### [Background Art]

Among cancers, the morbidity rate and mortality rate of bladder cancer have been on the rise (for example, refer to Non-Patent Document 1). Examples of a treatment method for bladder cancer include surgery, chemotherapy, and radiotherapy. However, the response rate for the treatment of bladder cancer is not favorable. Examples of the cause of this include the absence of useful cancer markers and follow-up markers.

### [Citation List]

### [Non-Patent Documents]

[Non-Patent Document 1]

Fitzmaurice C., et al., Global, Regional, and National Cancer Incidence, Mortality, Years of Life Lost, Years Lived With Disability, and Disability-Adjusted Life-Years for 29 Cancer Groups, 1990 to 2017: A Systematic Analysis for the Global Burden of Disease Study, JAMA Oncol., 5 (12), 1749-1768, 2019.

### [Summary of Invention]

### [Technical Problem]

An object of the present invention is to provide a technology for diagnosis of cancer.

### [Solution to Problem]

The present invention includes the following aspects.
[1] A kit for diagnosis of cancer, including: a specific binding substance to a SPRY domain-containing SOCS box protein 2 (SPSB2) protein; a primer set for amplifying cDNA of an SPSB2 gene; or a probe that specifically hybridizes to mRNA of an SPSB2 gene.
[2] The kit for diagnosis of cancer according to [1], in which the cancer is bladder cancer, pancreatic cancer, or hepatocellular carcinoma.
[3] A kit for determining a prognosis of a patient with cancer, including: a specific binding substance to an SPSB2 protein, a primer set for amplifying cDNA of an SPSB2 gene, or a probe that specifically hybridizes to mRNA of an SPSB2 gene.
[4] The kit for determining a prognosis according to [3], in which the cancer is bladder cancer, pancreatic cancer, or hepatocellular carcinoma.
[5] A method for determining a biological sample, including: a step of measuring an expression level of an SPSB2 protein or an SPSB2 gene in a biological sample, in which the fact that the measured expression level of the protein or gene is higher than that of a control indicates that the biological sample is derived from a patient with cancer.
[6] The determination method according to [5], in which the cancer is bladder cancer, pancreatic cancer, or hepatocellular carcinoma.
[7] A method for collecting data for determining whether or not a subject is affected by cancer, the method including: a step of measuring an expression level of an SPSB2 protein or an SPSB2 gene in a biological sample derived from the subject, in which the measured expression level of the protein or gene is the data for determining whether or not the subject is affected by cancer, with the previsio that medical practice by a doctor is excluded.
[8] The method according to [7], in which the cancer is bladder cancer, pancreatic cancer, or hepatocellular carcinoma.
[9] A method for predicting a prognosis of a patient with cancer, the method including: a step of measuring an expression level of an SPSB2 protein or an SPSB2 gene in a biological sample derived from the patient with cancer, in which the fact that the measured expression level of the protein or gene is higher than that of a control indicates that a prognosis of the patient with cancer is not favorable.
[10] The method according to [9], in which the cancer is bladder cancer, pancreatic cancer, or hepatocellular carcinoma.
[11] A method for screening an anti-cancer agent, the method including: a step of measuring an expression level of an SPSB2 protein or an SPSB2 gene in a cancer cell cultured in the presence of a test substance, in which the fact that the expression level is significantly decreased compared with an expression level of the SPSB2 protein or the SPSB2 gene in absence of the test substance indicates that the test substance is an anti-cancer agent.
[12] The method according [11], in which the cancer cell is a cancer cell derived from bladder cancer, pancreatic cancer, or hepatocellular carcinoma.

### [Advantageous Effects of Invention]

According to the present invention, it is possible to provide a new technology for diagnosis of cancer.

### [Brief Description of Drawings]

FIG. 1 is a graph showing a result of Experimental Example 2.
FIGS. 2(a) to 2(c) are photographs showing representative results of immunostaining in Experimental Example 3.
FIG. 3 is a graph showing a result of Experimental Example 5.
FIG. 4 is a graph showing a result of Experimental Example 5.
FIGS. 5(a) to 5(d) are photographs showing representative results of immunostaining in Experimental Example 6.
FIG. 6 is a graph showing a result of Experimental Example 7.
FIG. 7 is a graph showing a result of Experimental Example 7.
FIG. 8 is a photograph showing a result of the Western blotting in Experimental Example 8.
FIG. 9 is a photograph showing a result of the Western blotting in Experimental Example 8.
FIG. 10 is a graph showing quantitative values of an SPSB2 protein in a urine sample measured in Experimental Example 9.
FIG. 11 is a graph showing quantitative values of an SPSB2 protein in a urine sample measured in Experimental Example 9.
FIG. 12 is a graph showing quantitative values of an SPSB2 protein in a urine sample measured in Experimental Example 9.
FIG. 13 is a graph showing quantitative values of an SPSB2 protein in a urine sample measured in Experimental Example 9.
FIG. 14 is an ROC curve prepared in Experimental Example 10.
FIG. 15 is an ROC curve prepared in Experimental Example 10.
FIG. 16 is a graph showing results of analyzing a cancer-specific survival rate in Experimental Example 10.
FIG. 17 is a graph showing a result of analyzing a progression-free survival rate in Experimental Example 10.
FIG. 18(a) is a photograph showing a result of the Western blotting in Experimental Example 11. In addition, FIG. 18(b) is a graph showing a result of FIG. 18(a).

### [Description of Embodiments]

### [Kit for diagnosis of cancer, kit for determining prognosis of patient with cancer]

In one embodiment, the present invention provides a kit for diagnosis of cancer, including a substance that specifically binds to an SPSB2 protein, a primer set capable of amplifying cDNA of an SPSB2 gene, or a probe that specifically hybridizes with mRNA of an SPSB2 gene.

As will be described later in examples, the inventors clarified that the SPSB2 protein or the SPSB2 gene can be used as a cancer marker. In addition, the inventors clarified that the SPSB2 protein or the SPSB2 gene is useful as a marker not only for bladder cancer but also for pancreatic cancer and hepatocellular carcinoma. Therefore, in the kit for diagnosis of cancer of the present embodiment, examples of cancer include bladder cancer, pancreatic cancer, and hepatocellular carcinoma.

Using the kit of the present embodiment, it is possible to measure an expression level of an SPSB2 protein or an SPSB2 gene in a biological sample derived from a subject to determine whether or not the subject is affected by cancer. Examples of the biological sample include serum, plasma, urine, and tissue. In addition, particularly in a case where the cancer is bladder cancer, examples of the biological sample include urine, tissue, and the like. Here, with regard to urine, exosomes in urine may be extracted and used as a biological sample.

In addition, as will be described later in examples, the inventors analyzed patients with bladder cancer and patients with hepatocellular carcinoma and clarified that in a case where the SPSB2 protein or the SPSB2 gene is highly expressed, prognosis tends to be poor. Therefore, it can be also said that the kit for diagnosis of cancer of the present embodiment is a kit for determining prognosis. In the present specification, a poor prognosis may mean that the survival rate is low, the progression-free survival time is short, and the like.

That is, it can be said that the present invention provides a kit for determining a prognosis of a patient with cancer, including: a substance that specifically binds to an SPSB2 protein, a primer set capable of amplifying cDNA of an SPSB2 gene, or a probe that specifically hybridizes with mRNA of an SPSB2 gene. In the kit for determining a prognosis of a patient with cancer (kit for predicting prognosis of a patient with cancer), examples of cancer include bladder cancer, pancreatic cancer, and hepatocellular carcinoma.

NCBI accession numbers of human SPSB2 protein are NP_001139788.1, NP_001306599.1, NP_116030.1, and the like. In addition, NCBI accession numbers of mDNA of human SPSB2 gene are NM_001146316.2, NM_001146317.1, NM_001319670.2, NM_032641.4, and the like.

### [Substance that specifically binds]

The kit of the present embodiment may include a substance that specifically binds to an SPSB2 protein. Examples of the substance that specifically binds include an antibody, an antibody fragment, an aptamer, and the like. Examples of the antibody fragment include F(ab')₂, Fab', Fab, Fv, scFv, and the like. The antibody or antibody fragment may be polyclonal or monoclonal. The aptamer is not particularly limited as long as it has a specific binding ability to an SPSB2 protein, and examples thereof include a nucleic acid aptamer, a peptide aptamer, and the like.

For example, an expression level of the SPSB2 protein can be measured by performing immunostaining on an immobilized tissue section using the substance that specifically binds. The measurement of the expression level of the SPSB2 protein is not limited to immunostaining, and may be performed by extracting a protein from a test sample and using the Western blotting method or an ELISA method.

The fact that the expression level of the SPSB2 protein in the test sample is higher than that of a control indicates that the test sample is derived from a patient with cancer. In the present specification, "higher than that of a control" preferably means statistically significantly higher than that of a control. Here, examples of the control include an expression level of the SPSB2 protein measured using a sample derived from a normal tissue.

### (Primer set)

The kit for diagnosis of cancer of the present embodiment may include a primer set for amplifying cDNA of the SPSB2 gene. The sequence of the primer set is not particularly limited as long as it can amplify at least a part of cDNA of the SPSB2 gene.

The fact that the expression level of the SPSB2 gene in the test sample is higher than that of a control indicates that the test sample is derived from a patient with cancer. In addition, examples of the control include an expression level of an SPSB2 gene measured using a sample derived from a normal tissue.

### (Probe)

The kit of the present embodiment may include a probe that specifically hybridizes with mRNA of an SPSB2 gene.

The probe may be, for example, a nucleic acid fragment having a base sequence complementary to a base sequence of at least a part of mRNA of the SPSB2 gene. In addition, the probe may have various chemical modifications for the purpose of improving stability, specificity at the time of hybridization, and the like. For example, in order to prevent degradation by a hydrolase such as a nuclease, a phosphate residue may be substituted with a chemically modified phosphate residue such as phosphorothioate (PS), methylphosphonate, and phosphorodithionate. In addition, at least a part thereof may be composed of nucleic acid analogs such as peptide nucleic acid (PNA).

The probe may be fixed on a solid phase. Examples of the solid phase include a bead, a plate-like substrate, a film, and the like. The probe may be fixed on a surface of a plate-like substrate to form a microarray, for example. In this case, for example, an expression of the SPSB2 gene in the test sample can be detected by extracting RNA from the test sample, labeling the RNA with a fluorescent substance, and hybridizing the RNA with the microarray to detect the RNA binding to the probe on the microarray.

The fact that the expression level of the SPSB2 gene in the test sample is higher than that of a control indicates that the test sample is derived from a patient with cancer. Here, examples of the control include the expression level of the SPSB2 gene measured using the sample derived from a normal tissue.

### [Method for determining biological sample, method for collecting data for determining whether or not subject is affected by cancer, and method for predicting prognosis of patient with cancer]

In one embodiment, the present invention provides a method for determining a biological sample, including a step of measuring an expression level of an SPSB2 protein or gene in the biological sample, in which the fact that the measured expression level of the protein or gene is higher than that of a control indicates that the biological sample is derived from a patient with cancer.

As will be described later in examples, the inventors clarified that the SPSB2 protein or the SPSB2 gene can be used as a cancer marker. In addition, the inventors clarified that the SPSB2 protein or the SPSB2 gene is useful as a marker not only for bladder cancer but also for pancreatic cancer and hepatocellular carcinoma. Therefore, it is possible to determine whether or not a biological sample is derived from a patient with cancer by the method of the present embodiment. In the determination method of the present embodiment, examples of cancer include bladder cancer, pancreatic cancer, and hepatocellular carcinoma.

As described above, as the biological sample, it is possible to use serum, plasma, urine, tissue, and the like derived from a subject. In addition, particularly in a case where the cancer is bladder cancer, examples of the biological sample include urine, tissue, and the like. Here, with regard to urine, exosomes in urine may be extracted and used as a biological sample. In addition, similar to that described above, "higher than that of a control" preferably means statistically significantly higher than that of a control. In addition, examples of the control include the expression level of the SPSB2 protein or the SPSB2 gene, which is measured using a sample derived from a normal tissue.

It can also be said that the determination method of the present embodiment is a method for collecting data for determining whether or not a biological sample is derived from a patient with cancer. The method for collecting data does not include medical practice by a doctor.

That is, it can be said that the present invention provides a method for collecting data for determining whether or not a biological sample is derived from a patient with cancer, the method including a step of measuring an expression level of an SPSB2 protein or gene in the biological sample, in which the measured expression level of the protein or gene is data for determining whether or not the biological sample is derived from a patient with cancer. The fact that the expression level of the SPSB2 protein or gene in the biological sample is higher than that of a control indicates that the biological sample is derived from a patient with cancer.

Alternatively, it can be said that the present invention provides a method for collecting data for determining whether or not a subject is affected by cancer, the method including a step of measuring an expression level of an SPSB2 protein or gene in a biological sample derived from the subject, in which the measured expression level of the protein or gene is data for determining whether or not the subject is affected by cancer. The fact that the expression level of the SPSB2 protein or gene in the biological sample derived from the subject is higher than that of a control indicates that the subject is affected by cancer. In the method for collecting data, examples of cancer include bladder cancer, pancreatic cancer, and hepatocellular carcinoma.

In addition, as will be described later in examples, the inventors analyzed patients with bladder cancer and patients with hepatocellular carcinoma and clarified that in a case where the SPSB2 protein or the SPSB2 gene is highly expressed, the prognosis tends to be poor. Therefore, it can be said that the determination method of the present embodiment is a method for predicting a prognosis.

That is, it can be said that the present invention provides a method for predicting a prognosis of a patient with cancer, the method including a step of measuring an expression level of an SPSB2 protein or an SPSB2 gene in the biological sample derived from a patient with cancer, in which the fact that the measured expression level of the protein or gene is higher than that of a control indicates that the prognosis of the patient with cancer is not favorable. In the method for predicting a prognosis of a patient with cancer, examples of cancer include bladder cancer, pancreatic cancer, and hepatocellular carcinoma. [0031]

### [Method for screening anti-cancer agent]

In one embodiment, the present invention provides a method for screening an anti-cancer agent, the method including a step of measuring an expression level of an SPSB2 protein or an SPSB2 gene in a cancer cell cultured in the presence of a test substance, in which the fact that the expression level is significantly decreased compared with an expression level of the SPSB2 protein or the SPSB2 gene in the absence of the test substance indicates that the test substance is an anti-cancer agent.

As will be described later in examples, the higher a malignant degree of a cancer cell, the higher the expression level of the SPSB2 protein or SPSB2 gene tends to be. Therefore, it can be said that a test substance that decreases the expression level of the SPSB2 protein or the SPSB2 gene is a candidate substance of an anti-cancer agent.

In the screening method of the present embodiment, the test substance is not particularly limited, and examples thereof include a natural compound library, a synthetic compound library, an existing drug library, a metabolite library, and the like.

In the screening method of the present embodiment, the cancer cell may be a cancer cell derived from bladder cancer, pancreatic cancer, hepatocellular carcinoma, and the like.

In addition, the cancer cell may be a cisplatin-resistant strain obtained by culturing in the presence of cisplatin while gradually increasing the concentration of cisplatin. The cisplatin-resistant strain tends to be resistant to an anti-cancer agent other than cisplatin and tends to have a high malignant degree.

### [Other embodiments]

In one embodiment, the present invention provides a method for treating cancer, including a step of measuring an expression level of an SPSB2 protein or an SPSB2 gene in a biological sample derived from a subject, in which the fact that the expression level is higher than that of a control indicates that the subject is affected by cancer; and a step of excising a cancer tissue from a subject by surgical operation or performing anti-cancer agent treatment on the subject in a case where the subject is affected by cancer.

In the treatment method of the present embodiment, examples of the cancer include bladder cancer, pancreatic cancer, hepatocellular carcinoma, and the like. In addition, examples of the biological sample include serum, plasma, urine, tissue, and the like. In particular, in a case where the cancer is bladder cancer, examples of the biological sample include urine, tissue, and the like. Here, with regard to urine, exosomes in urine may be extracted and used as a biological sample.

As will be described later in examples, in a case where urine is used as a biological sample, there is a case where the expression level of the SPSB2 protein or the SPSB2 gene may be high not only in a patient with cancer but also in a patient with a urinary tract infection. In this case, it is possible to diagnose whether or not a subject has a urinary tract infection by a urine culture test and the like. That is, a subject with a high expression level of the SPSB2 protein or the SPSB2 gene and without a urinary tract infection can be diagnosed as being affected by cancer.

In the treatment method of the present embodiment, examples of the anti-cancer agent include cisplatin, M-VAC (combination of methotrexate, vinblastine, adriamycin, and cisplatin), GC (combination of cisplatin and Gemzar), enfortumab vedotin which is an antibody-drug complex (ADC) targeting nectin-4, Padcev, Keytruda (pembrolizumab), and the like.

These anti-cancer agents are generally administered by intravenous drip infusion. In addition, the dosage of these anti-cancer agents varies depending on symptoms, body weight, age, gender, and the like of the patient, but an appropriate dosage can be appropriately selected by those skilled in the art.

### [Examples]

Next, the present invention will be described in more detail by showing experimental examples, but the present invention is not limited to the experimental examples below.

### [Experimental Example 1]

The inventors have already prepared a large number of bladder cancer-specific antibodies in studies up to now. In addition, a technology for identifying a target protein using an autoantibody in the serum by a dot blot method has been already established, and the identification of a large number of autoantibodies against bladder cancer has been achieved. Reactivity of these prepared bladder cancer-specific antibodies and autoantibodies against bladder cancer to a large number of collected examples of bladder cancer patient serum and tumor tissue was examined. As a result, the SPSB2 protein or the SPSB2 gene was specified as a candidate for a new cancer marker.

### [Experimental Example 2]

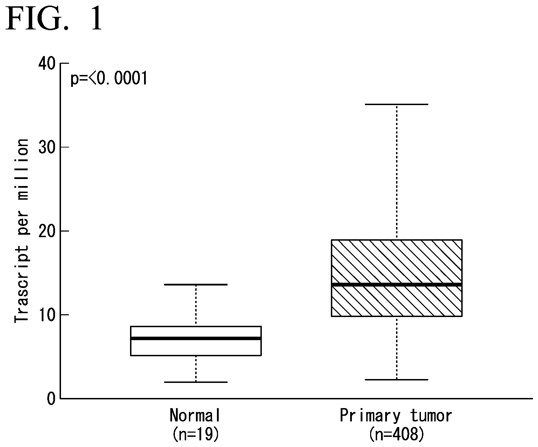

The expression level of the SPSB2 gene in the bladder cancer tissue was examined using The Cancer Genome Atlas (TCGA), which is a public database. FIG. 1 is a graph showing examination results. In FIG. 1, the vertical axis represents the expression level of the SPSB2 gene. In addition, "Normal" indicates the expression level of the SPSB2 gene in a normal tissue, and "Primary tumor" indicates the expression level of the SPSB2 gene in the bladder cancer tissue. As a result, it was clarified that the expression level of the SPSB2 gene was significantly increased in the bladder cancer tissue.

### [Experimental Example 3]

Tissue sections prepared from total cystectomized specimens were immunostained with an anti-SPSB2 antibody. As the total cystectomized specimens, 126 cases of specimens on which total cystectomy was performed at Kitasato University Hospital from 1990 to 2015 became subjects. ABond-MAX automatic immunostaining device (Leica Biosystems) was used for immunostaining.

The intensity of nuclear staining of tumor cells with the anti-SPSB2 antibody in the tissue section was compared with the intensity of nuclear staining of surrounding cells with the anti-SPSB2 antibody, and the intensity was evaluated in three stages according to the following evaluation criteria. The evaluation criteria 0 to 1 were classified into the SPSB2 protein low expression group, and the evaluation criteria 2 were classified into the SPSB2 protein high expression group to perform the following analysis.

### (Evaluation criteria)

0: Low expression
1: Equivalent
2: High expression

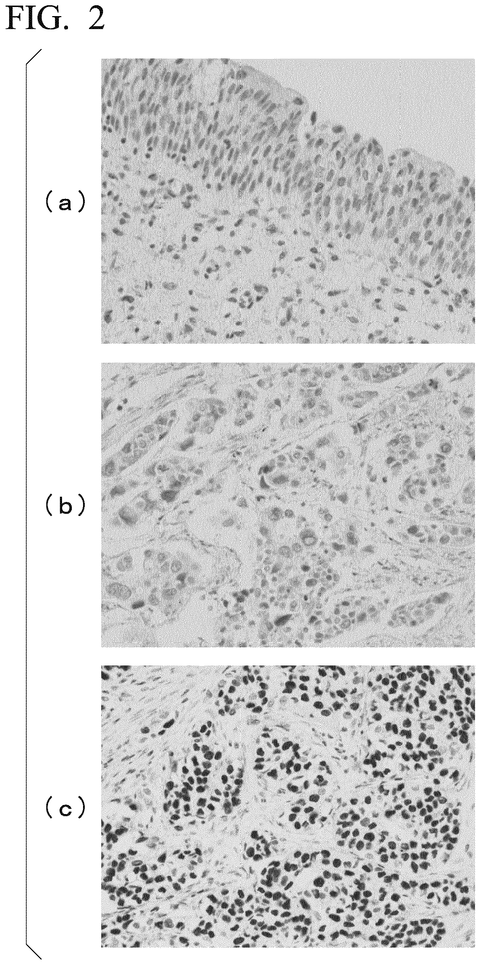

FIGS. 2(a) to 2(c) are photographs showing results of representative immunostaining. FIG. 2(a) is a photograph showing results of immunostaining of a normal urothelial tissue. In addition, FIG. 2(b) is a photograph showing results of immunostaining of a bladder cancer tissue classified into an SPSB2 protein low expression group. In addition, FIG. 2(c) is a photograph showing results of immunostaining of a bladder cancer tissue classified into an SPSB2 protein high expression group.

### [Experimental Example 4]

Based on the results of Experimental Example 3, the relationship between the expression of the SPSB2 protein and clinical pathological factors was analyzed. The analysis results are shown in Table 1 below. In Table 1, "p value" indicates a p value calculated by Fisher's exact test. p < 0.05 was determined to be significantly different. Bold letters indicate that there is a significant difference. As a result, it was clarified that the expression of the SPSB2 protein is correlated with gender, degree of vertical invasion (pT stage), degree of atypia, and vascular invasion.

**[Table 1]**

| | Number of patients (%) | Expression of SPSB2 protein | | |
|---|---|---|---|---|
| | | Low expression group | High expression group | p value |
| Sum (%) | 126 | 67 (53.2) | 59 (46.8) | |

| Age | | | | |
|---|---|---|---|---|
| ≤65 | 66 (52.4) | 37 (56.1) | 29 (43.9) | |
| >65 | 60 (47.6) | 30 (50.0) | 30 (50.0) | 0.592 |

| Gender | | | | |
|---|---|---|---|---|
| Male | 101 (80.2) | 59 (58.4) | 42 (41.6) | |
| Female | 25 (19.8) | 8 (32.0) | 17 (68.0) | 0.025 |
| pT stage | | | | |
| ≤pT1 | 26 (20.6) | 21 (80.8) | 5 (19.2) | |
| ≥pT2 | 100 (79.4) | 46 (46.0) | 54 (54.0) | 0.002 |

| Degree of atypia | | | | |
|---|---|---|---|---|
| Grades 1 and 2 | 37 (29.6) | 26 (70.3) | 11 (29.7) | |
| Grade 3 | 88 (70.4) | 41 (46.6) | 47 (53.4) | 0.019 |

| Carcinoma in situ | | | | |
|---|---|---|---|---|
| Absent | 108 (85.7) | 57 (52.8) | 51 (47.2) | |
| Present | 18 (14.3) | 10 (55.6) | 8 (44.4) | >0.99 |

| Vascular invasion | | | | |
|---|---|---|---|---|
| Absent | 42 (37.2) | 31 (73.8) | 11 (26.2) | |
| Present | 71 (62.8) | 31 (43.7) | 40 (56.3) | 0.003 |

| Lymph node metastasis | | | | |
|---|---|---|---|---|
| N0 | 93 (77.5) | 53 (57.0) | 40 (43.0) | |
| N1 or N2 | 27 (22.5) | 12 (44.4) | 15 (55.6) | 0.279 |

Table 2 below shows the results of examining the correlation between the expression of S 100A8 protein, S100A9 protein, Uroplakin III protein, and HNRNPA3 protein, which have been reported to be bladder cancer markers, and the expression of SPSB2 protein. In Table 2, "p value" indicates a p value calculated by Fisher's exact test. p < 0.05 was determined to be significantly different. Bold letters indicate that there is a significant difference.

As a result, it was clarified that the expression of the SPSB2 protein is correlated with the expression of each of the S 100A8 protein, the S100A9 protein, the Uroplakin III protein, and the HNRNPA3 protein.

**[Table 2]**

| | Number of patients (%) | Expression of SPSB2 protein | | |
|---|---|---|---|---|
| | | Low expression group | High expression group | p value |
| | 126 | 67 (53.2) | 59 (46.8) | |

| Expression of S100A8 | | | | |
|---|---|---|---|---|
| Normal | 49 (62.0) | 30 (61.2) | 19 (38.8) | |
| Abnormal | 30 (38.0) | 10 (33.3) | 20 (66.7) | **0.021** |
| Expression of S100A9 | | | | |
| Normal | 54 (68.4) | 32 (59.3) | 22 (40.7) | |
| Abnormal | 25 (31.6) | 8 (32.0) | 17 (68.0) | **0.031** |

| Expression of Uroplakin III | | | | |
|---|---|---|---|---|
| Absent | 51 (64.6) | 23 (45.1) | 28 (54.9) | |
| Present | 28 (35.4) | 20 (71.4) | 8 (28.6) | **0.034** |

| Expression of HNRNPA3 | | | | |
|---|---|---|---|---|
| Low expression | 85 (69.7) | 54 (63.5) | 31 (36.5) | |
| High expression | 37 (30.3) | 11 (29.7) | 26 (70.3) | **0.001** |

### [Experimental Example 5]

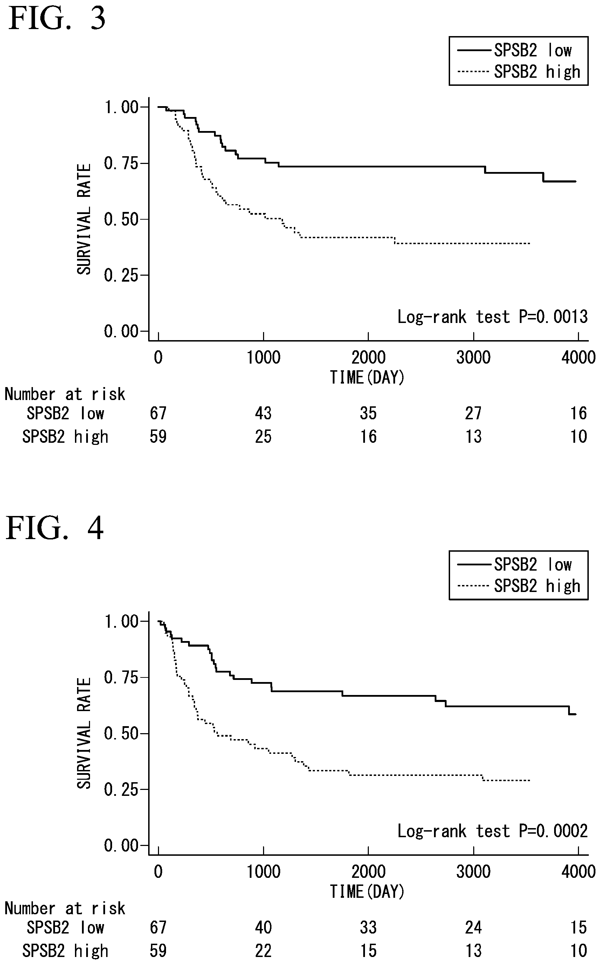

Based on the results of Experimental Example 3, the relationship between the expression of the SPSB2 protein and prognosis was analyzed. FIG. 3 is a graph showing results of analyzing the cancer-specific survival rate by the Kaplan-Meier method. In FIG. 3, "SPSB2 low" indicates results for the SPSB2 protein low expression group, and "SPSB2 high" indicates results for the SPSB2 protein high expression group. In addition, "Number at risk" indicates the number of survivors at each time point. As a result, it was clarified that the SPSB2 protein high expression group had a significantly high mortality risk due to bladder cancer.

FIG. 4 is a graph showing results of analyzing the progression-free survival rate by the Kaplan-Meier method based on the results of Experimental Example 3. In FIG. 4, "SPSB2 low" indicates results for the SPSB2 protein low expression group, and "SPSB2 high" indicates results for the SPSB2 protein high expression group. In addition, "Number at risk" indicates the number of progression-free survivors at each time point. As a result, it was clarified that a period of time until bladder cancer recurrence was significantly short in the SPSB2 protein high expression group.

Table 3 below shows the results of univariate analysis and multivariate analysis based on a Cox proportional hazard model, regarding the cancer-specific survival rate. In addition, Table 4 below shows results of performing univariate analysis and multivariate analysis based on the Cox proportional hazard model, regarding the progression-free survival rate. In Tables 3 and 4, "HR" indicates a hazard ratio, and "95% CI" indicates 95% confidence interval. p < 0.05 was determined to be significantly different. Bold letters indicate that there is a significant difference.

As a result, it was clarified that the expression of the SPSB2 protein was an independent factor of cancer-specific survival rate and progression-free survival rate together with lymph node metastasis.

**[Table 3]**

| | Cancer-specific survival rate | | | | | |
|---|---|---|---|---|---|---|
| | Univariate analysis | | | Multivariate analysis | | |
| | HR | 95% CI | p value | HR | 95% CI | p value |
| Expression of SPSB2 | 2.47 | 1.40-4.37 | **0.002** | 2.26 | 1.15-4.44 | **0.018** |
| Pathological stage | 2.01 | 0.98-4.13 | 0.057 | 1.42 | 0.50-4.06 | 0.514 |
| Lymph node metastasis | 3.03 | 1.69-5.45 | **<0.001** | 2.76 | 1.38-5.52 | **0.004** |
| Vascular invasion | 2.43 | 1.23-4.80 | **0.010** | 1.08 | 0.47-2.47 | 0.860 |
| Degree of atypia | 1.65 | 0.87-3.15 | 0.128 | 1.13 | 0.51-2.49 | 0.772 |
| Carcinoma in situ | 0.43 | 0.15-1.18 | 0.101 | 0.43 | 0.13-1.44 | 0.172 |

**[Table 4]**

| | Progression-free survival rate | | | | | |
|---|---|---|---|---|---|---|
| | Univariate analysis | | | Multivariate analysis | | |
| | HR | 95% CI | p value | HR | 95% CI | p value |
| Expression of SPSB2 | 2.58 | 1.54-4.33 | **<0.001** | 2.10 | 1.14-3.86 | **0.017** |
| Pathological stage | 2.24 | 1.14-4.41 | **0.019** | 1.58 | 0.61-4.08 | 0.348 |
| Lymph node metastasis | 2.86 | 1.67-4.89 | **<0.001** | 2.29 | 1.21-4.34 | **0.011** |
| Vascular invasion | 2.52 | 1.35-4.71 | **0.004** | 1.32 | 0.62-2.78 | 0.472 |
| Degree of atypia | 1.47 | 0.83-2.60 | 0.182 | 1.05 | 0.51-2.16 | 0.897 |
| Carcinoma in situ | 0.64 | 0.29-1.41 | 0.273 | 0.87 | 0.36-2.12 | 0.766 |

### [Experimental Example 6]

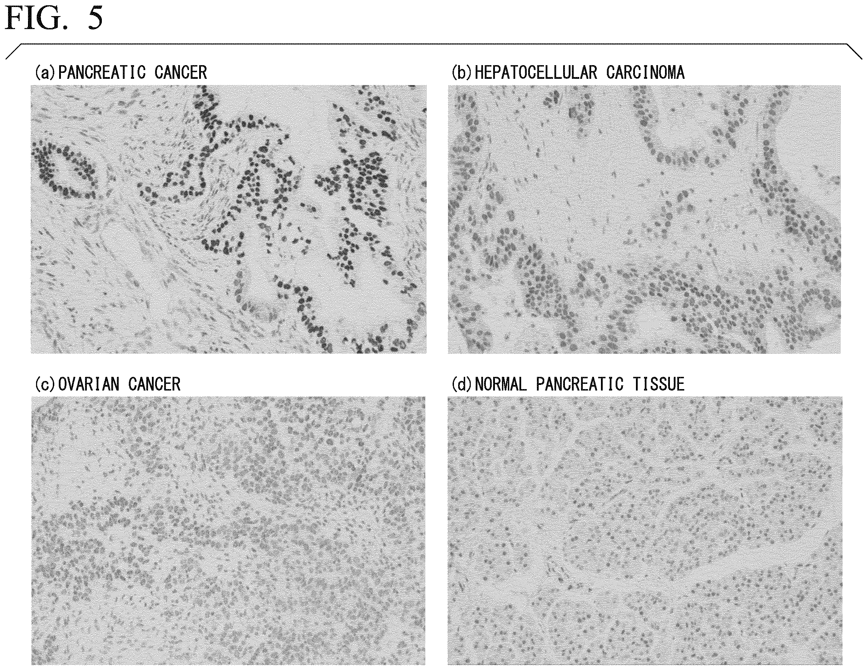

The expression of the SPSB2 protein in cancer types other than bladder cancer was examined. FIGS. 5(a) to 5(c) are photographs showing representative results of immunostaining of each tissue section of pancreatic cancer, hepatocellular carcinoma, and ovarian cancer with an anti-SPSB2 antibody. FIG. 5(a) is a result of the tissue section of pancreatic cancer, FIG. 5(b) is a result of the tissue section of hepatocellular carcinoma, FIG. 5(c) is a result of the tissue section of ovarian cancer, and FIG. 5(d) is a result of the tissue section of the normal tissue of the pancreas.

As a result, it was clarified that the SPSB2 protein was acknowledged to be strongly expressed in pancreatic cancer and acknowledged to be moderately expressed in hepatocellular carcinoma and ovarian cancer. On the other hand, the SPSB2 protein was weakly expressed or not expressed in kidney cancer, prostate cancer, esophageal cancer, gastric cancer, colon cancer, breast cancer, and lung cancer.

### [Experimental Example 7]

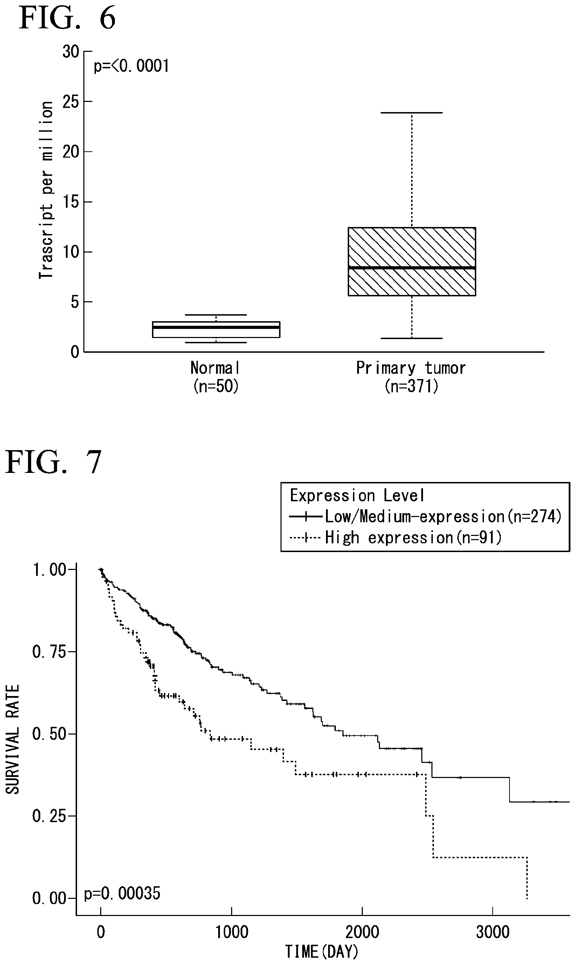

The expression level of the SPSB2 gene in hepatocellular carcinoma tissue was examined using The Cancer Genome Atlas (TCGA), which is a public database. FIG. 6 is a graph showing examination results. In FIG. 6, the vertical axis indicates the expression level of the SPSB2 gene. In addition, "Normal" indicates the expression level of the SPSB2 gene in a normal tissue, and "Primary tumor" indicates the expression level of the SPSB2 gene in hepatocellular carcinoma tissue.

As a result, it was clarified that the expression level of the SPSB2 gene is significantly increased in the hepatocellular carcinoma tissue.

FIG. 7 is a graph showing the relationship between the expression level of the SPSB2 gene in hepatocellular carcinoma and prognosis in TCGA samples. In FIG. 7, "High expression" indicates results for the SPSB2 protein high expression group, and "Low/Medium-expression" indicates results for the SPSB2 protein low to medium expression groups. As a result, it was clarified that the SPSB2 gene high expression group of hepatocellular carcinoma has a significantly poor prognosis.

### [Experimental Example 8]

The abundance of the SPSB2 protein in serum exosomes and urinary exosomes derived from a healthy subject and a patient with bladder cancer was examined by the Western blotting.

First, exosomes were extracted from serum samples and urine samples using Total Exosome Isolation Reagent (Thermo Fisher Scientific, Inc.).

Subsequently, the SPSB2 protein was detected using the extracted exosomes. In addition, it was confirmed that exosomes could be extracted by the Western blotting of CD9, which is one of the exosome markers. A molecular weight of the SPSB2 protein is about 26 kDa, and a molecular weight of CD9 is about 24 kDa.

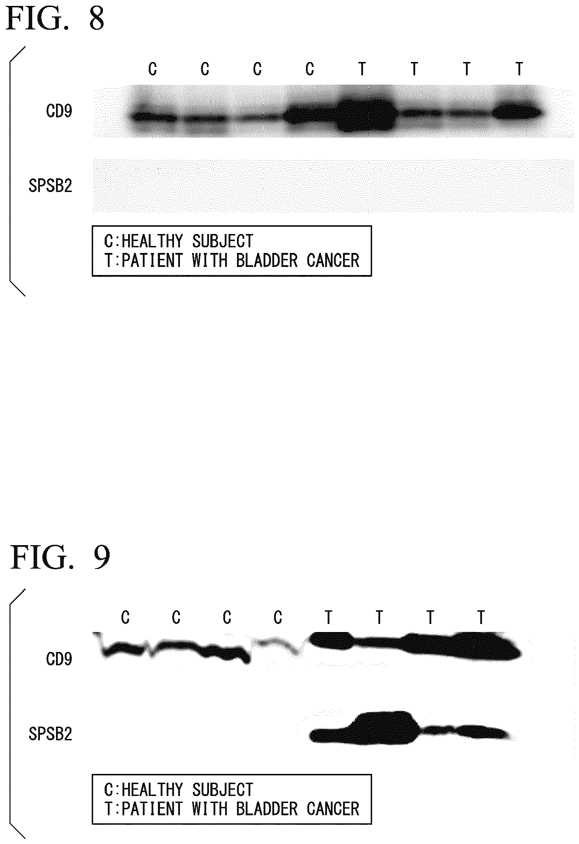

FIG. 8 is a photograph showing results of the Western blotting on exosomes in serum. In FIG. 8, "C" indicates exosomes derived from a healthy subject, and "T" indicates exosomes derived from a patient with bladder cancer.

As a result, it was confirmed that exosomes could be extracted since CD9 could be detected. In addition, it was also clarified that the presence of the SPSB2 protein was not acknowledged in exosomes in serum in both of the healthy subject and the patient with bladder cancer.

FIG. 9 is a photograph showing results of the Western blotting on exosomes in urine. In FIG. 9, "C" indicates exosomes derived from a healthy subject, and "T" indicates exosomes derived from a patient with bladder cancer.

As a result, it was confirmed that exosomes could be extracted since CD9 could be detected. In addition, the presence of the SPSB2 protein was not acknowledged in exosomes in serum derived from a healthy subject, whereas the presence of the SPSB2 protein was detected in the urinary exosomes derived from a patient with bladder cancer.

### [Experimental Example 9]

The abundances of the SPSB2 protein in urine derived from a healthy subject, a patient with urinary calculi, a patient with urinary tract infection, and a patient with bladder cancer were quantified by enzyme-linked immunosorbent assay (ELISA) and examined.

As patients with bladder cancer, 91 cases in which urine was collected immediately before transurethral bladder tumor resection at Kitasato University Hospital from 2009 to 2015 became subjects. The background of patients with bladder cancer is shown in Table 5 below. In Table 5, "NMIBC" indicates non-muscle-invasive bladder cancer, and "MIBC" indicates muscle-invasive bladder cancer. Urine specific gravity of all urine samples was corrected to 1.002, and the abundance of the SPSB2 protein was measured by ELISA.

**[Table 5]**

| | Number of patients (%) |
|---|---|
| Sum (%) | 91 |

| Expression of SPSB2 (ng/mL) | |
|---|---|
| Median value | 221.39 |
| Range | 0.14 to 3468.40 |

| Age | |
|---|---|
| Median value | 75 |
| Range | 43 to 90 |

| Gender | |
|---|---|
| Male | 67 (65.9) |
| Female | 24 (26.4) |

| pT stage | |
|---|---|
| NMIBC | 60 (65.9) |
| MIBC | 31 (34.1) |

| Pathological grade | |
|---|---|
| Grades 1 and 2 | 46 (50.5) |
| Grade 3 | 44 (48.4) |
| Unclear | 1 (1.1) |

| Urine cytology | |
|---|---|
| Negative | 39 (42.9) |
| Positive | 49 (53.8) |
| Unclear | 3 (3.3) |

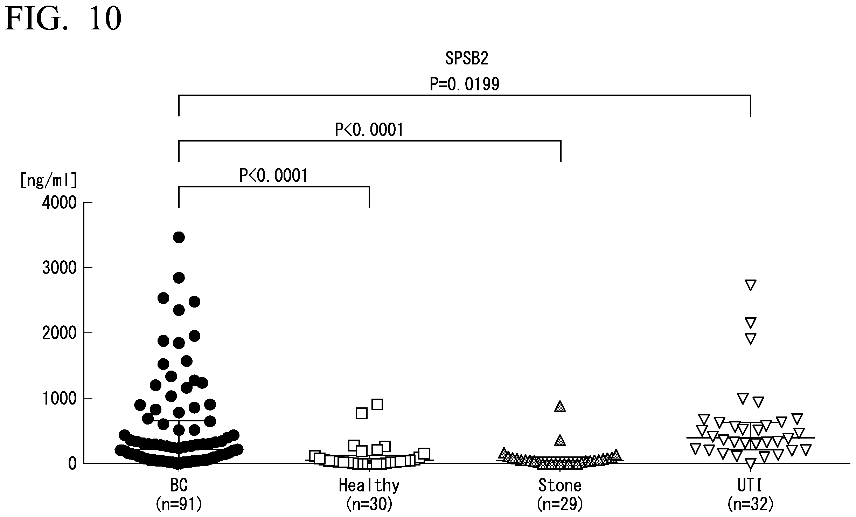

FIG. 10 is a graph showing quantitative values of the SPSB2 protein in urine samples of each group. In addition, FIG. 10 also shows analysis results by the Mann-Whitney U-test. In FIG. 10, "BC" indicates results of a patient with bladder cancer, "Healthy" indicates results of a healthy subject, "Stone" indicates results of a patient with urinary calculi, and "UTI" indicates results of a patient with urinary tract infection.

As a result, the abundance of the SBSP2 protein in urine of the patient with bladder cancer was higher than those of the healthy subject and the patient with urinary calculi, but was lower than that of the patient with urinary tract infection. The urinary tract infection can be diagnosed by a urine culture test and the like.

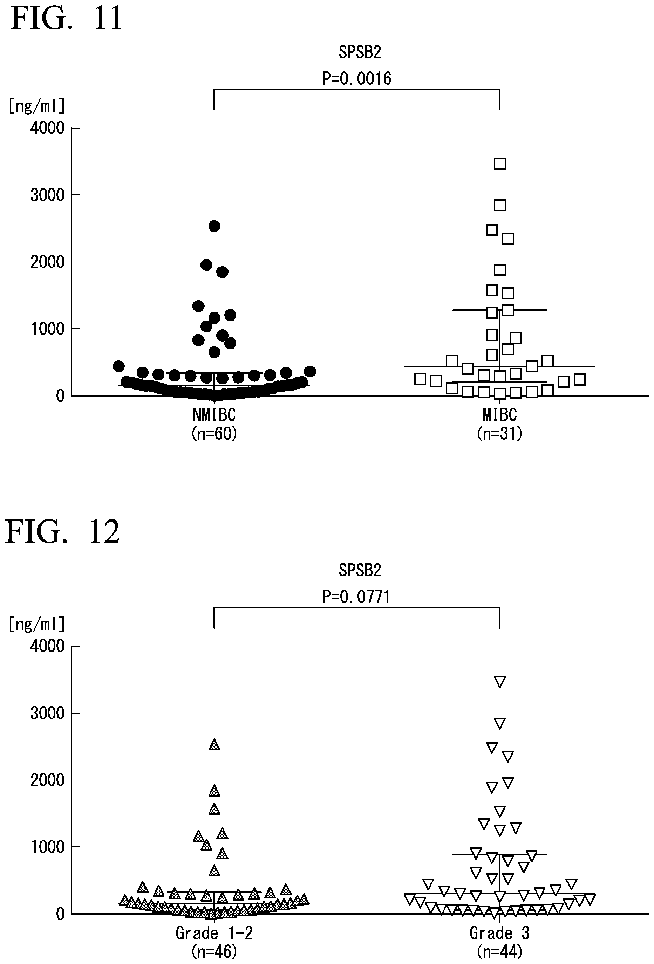

FIG. 11 is a graph showing quantitative values of the SPSB2 protein in urine samples of patients with non-muscle-invasive bladder cancer and patients with muscle-invasive bladder cancer. In addition, FIG. 11 also shows the analysis results by the Mann-Whitney U-test. In FIG. 11, "NMIBC" indicates non-muscle-invasive bladder cancer, and "MIBC" indicates muscle-invasive bladder cancer.

As a result, it was clarified that the abundance of the SBSP2 protein in urine of patients with bladder cancer shows a higher value than that of the muscle-invasive bladder cancer.

FIG. 12 is a graph showing quantitative values of the SPSB2 protein in urine samples of patients with bladder cancer of pathological grades 1 and 2 and patients with bladder cancer of pathological grade 3. In addition, FIG. 12 also shows the analysis results by the Mann-Whitney U-test.

As a result, it was clarified that the abundance of the SBSP2 protein in urine of the patients with bladder cancer of pathological grade 3 tends to show a higher value than that of the patients with bladder cancer of pathological grades 1 and 2.

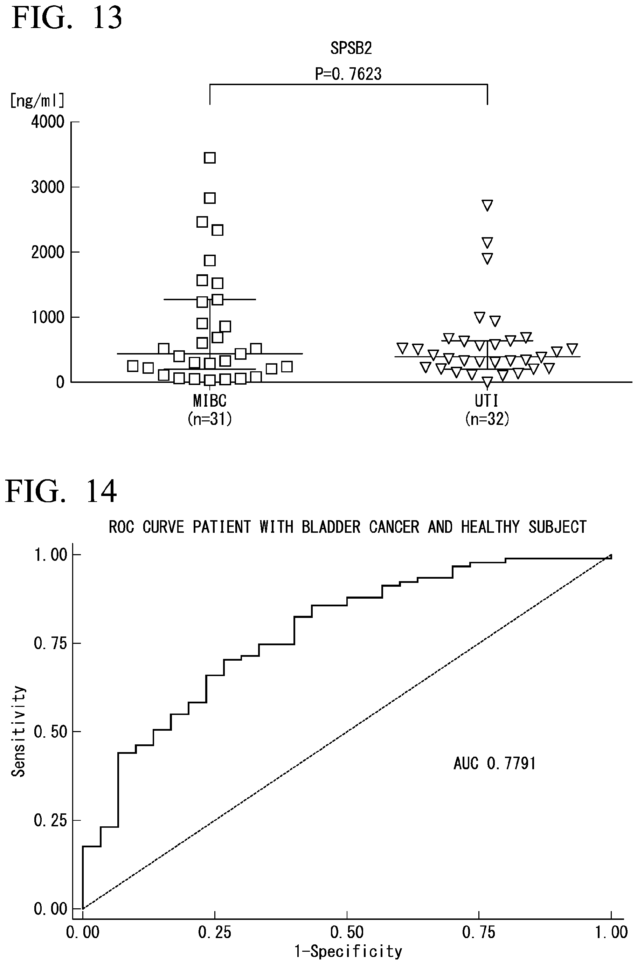

FIG. 13 is a graph showing quantitative values of the SPSB2 protein in urine samples of patients with muscle-invasive bladder cancer and patients with urinary tract infection. In addition, FIG. 13 also shows the analysis results by the Mann-Whitney U-test. In FIG. 13, "MIBC" indicates muscle-invasive bladder cancer, and "UTI" indicates results of patients with urinary tract infection.

As a result, no significant difference was acknowledged in the abundance of the SBSP2 protein in urine of patients with muscle-invasive bladder cancer and urinary tract infection. The urinary tract infection can be diagnosed by a urine culture test and the like.

### [Experimental Example 10]

Based on the results of Experimental Example 9, the relationship between the expression of the SPSB2 protein and prognosis was analyzed. FIG. 14 is an ROC curve prepared based on the quantitative values of the SPSB2 protein in urine samples of patients with bladder cancer and healthy subjects, which were measured in Experimental Example 9. As a result, it was clarified that the area under the ROC curve (AUC: area under the curve) is 0.7791.

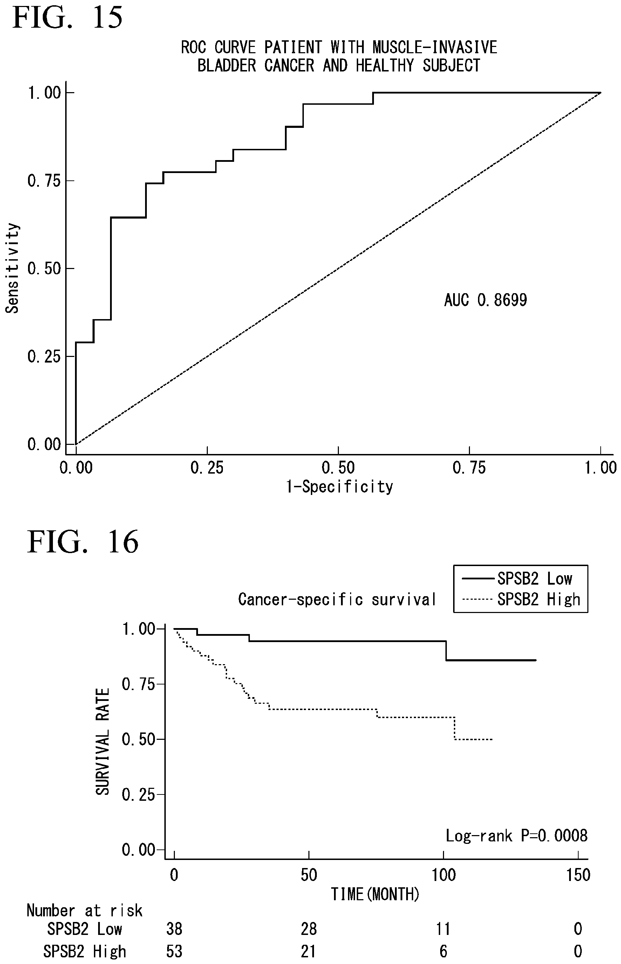

FIG. 15 is an ROC curve prepared based on the quantitative values of the SPSB2 protein in urine samples of patients with muscle-invasive bladder cancer and healthy subjects, which were measured in Experimental Example 9. As a result, it was clarified that the area under the ROC curve (AUC) is 0.8699.

Subsequently, based on the ROC curve, a cutoff value was set to 162.8 ng/mL (sensitivity: 58.2%, specificity: 80.0%), and survival analysis was performed, with those having the abundance of the SPSB2 protein in urine of less than 162.8 ng/mL as an SPSB2 protein low expression group and those having the abundance of the SPSB2 protein in urine of 162.8 ng/mL or more as an SPSB2 protein high expression group.

FIG. 16 is a graph showing results of analyzing the cancer-specific survival rate by the Kaplan-Meier method. In FIG. 16, "SPSB2 Low" indicates results for the SPSB2 protein low expression group, and "SPSB2 High" indicates results for the SPSB2 protein high expression group. In addition, "Number at risk" indicates the number of survivors at each time point. As a result, it was clarified that the SPSB2 protein high expression group had a significantly high mortality risk due to bladder cancer.

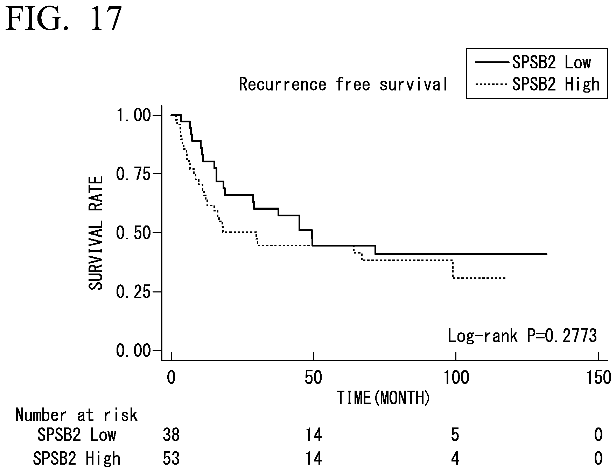

FIG. 17 is a graph showing results of analyzing the progression-free survival rate by the Kaplan-Meier method. In FIG. 17, "SPSB2 Low" indicates results for the SPSB2 protein low expression group, and "SPSB2 High" indicates results for the SPSB2 protein high expression group. In addition, "Number at risk" indicates the number of progression-free survivors at each time point. As a result, no significant difference was acknowledged in the period of time until bladder cancer recurrence between the SPSB2 protein low expression group and the SPSB2 protein high expression group.

Table 6 below shows results of univariate analysis and multivariate analysis based on the Cox proportional hazard model, regarding cancer-specific survival rate. In Table 6, "NMIBC" indicates non-muscle-invasive bladder cancer, "MIBC" indicates muscle-invasive bladder cancer, "G3" indicates Grade 3, and "G1, 2" indicates Grade 1 or 2, "HR" indicates a hazard ratio, and "95% CI" indicates a 95% confidence interval. p < 0.05 was determined to be significantly different. Bold letters indicate that there is a significant difference.

As a result, it was clarified that the expression of the SPSB2 protein is a prognostic factor of cancer-specific survival rate together with the degree of vertical invasion of cancer.

**[Table 6]**

| | | Cancer-specific survival rate | | | | | |
|---|---|---|---|---|---|---|---|
| | | Univariate analysis | | | Multivariate analysis | | |
| Variable | Category | HR | 95% CI | p value | HR | 95% CI | p value |
| Expression of SPSB2 | High vs. Low | 6.23 | 1.84-21.17 | **0.003** | 4.54 | 1.31-15.77 | **0.017** |
| Pathological stage | MIBC vs. NMIBC | 5.83 | 2.37-14.34 | **<0.001** | 4.40 | 1.65-11.75 | **0.003** |
| Pathological grade | G3 vs. G1, 2 | 2.58 | 1.05-6.34 | 0.039 | 1.06 | 0.35-3.18 | 0.917 |
| Urine cytology | Positive vs. Negative | 2.64 | 1.02-6.86 | 0.047 | 1.61 | 0.47-4.50 | 0.367 |

### [Experimental Example 11]

T24 and 5637, which are human bladder cancer cell lines, were cultured in the presence of cisplatin while gradually increasing the concentration to obtain T24CDDPR and 5637CDDPR, each of which are cisplatin-resistant strains. T24CDDPR and 5637CDDPR are considered to have a higher degree of malignant cancer than T24 and 5637.

Subsequently, the expression level of the SPSB2 protein was measured by the Western blotting of each cell line of T24, T24CDDPR, 5637, and 5637CDDPR. In addition, the expression level of β-actin protein was measured as a control.

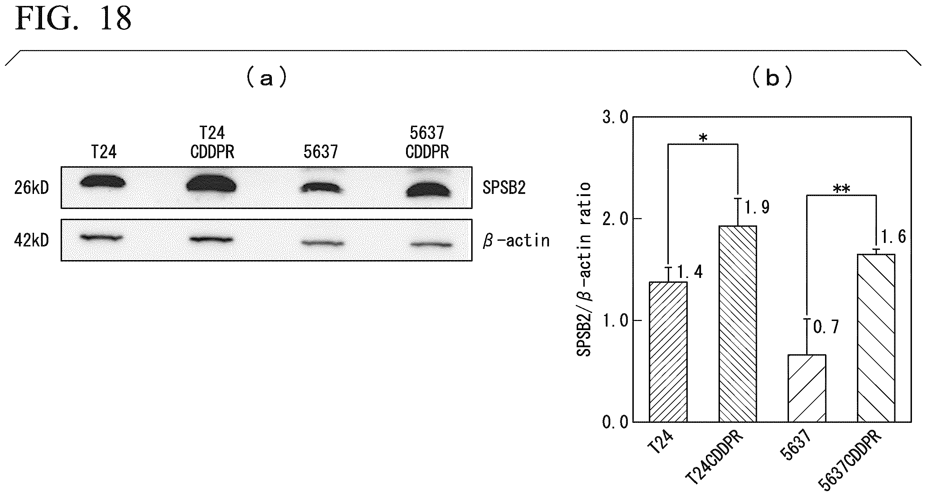

FIG. 18(a) is a photograph showing results of the Western blotting. In addition, FIG. 18(b) shows the results of FIG. 18(a) in the form of a graph. The vertical axis in FIG. 18(b) indicates the expression level of the SPSB2 with respect to the expression level of β-actin, "*" indicates that there is a significant difference at p < 0.05, and "**" indicates that there is a significant difference at p < 0.01.

As a result, it was clarified that the expression level of the SPSB2 is significantly higher in T24CDDPR and 5637CDDPR than in each of T24 and 5637.

This result indicates that the higher the malignant degree of cancer, the higher the expression level of the SPSB2 in cancer cells.

### [Industrial Applicability]

According to the present invention, it is possible to provide a new technology for diagnosis of cancer.

## Claims

1. A kit for diagnosis of cancer, comprising:
a specific binding substance to a SPRY domain-containing SOCS box protein 2 (SPSB2) protein;
a primer set for amplifying cDNA of an SPSB2 gene; or
a probe that specifically hybridizes to mRNA of an SPSB2 gene.

2. The kit for diagnosis of cancer according to Claim 1, wherein the cancer is bladder cancer, pancreatic cancer, or hepatocellular carcinoma.

3. A kit for determining a prognosis of a patient with cancer, comprising:
a specific binding substance to an SPSB2 protein;
a primer set for amplifying cDNA of an SPSB2 gene; or
a probe that specifically hybridizes to mRNA of an SPSB2 gene.

4. The kit for determining a prognosis according to Claim 3, wherein the cancer is bladder cancer, pancreatic cancer, or hepatocellular carcinoma.

5. A method for determining a biological sample, comprising:
a step of measuring an expression level of an SPSB2 protein or an SPSB2 gene in a biological sample,
wherein the fact that the measured expression level of the protein or gene is higher than that of a control indicates that the biological sample is derived from a patient with cancer.

6. The method for determining according to Claim 5, wherein the cancer is bladder cancer, pancreatic cancer, or hepatocellular carcinoma.

7. A method for collecting data for determining whether or not a subject is affected by cancer, the method comprising: a step of measuring an expression level of an SPSB2 protein or an SPSB2 gene in a biological sample derived from the subject, wherein the measured expression level of the protein or gene is the data for determining whether or not the subject is affected by cancer, with the previsio that medical practice by a doctor is excluded.

8. The method according to Claim 7, wherein the cancer is bladder cancer, pancreatic cancer, or hepatocellular carcinoma.

9. A method for predicting a prognosis of a patient with cancer, the method comprising: a step of measuring an expression level of an SPSB2 protein or an SPSB2 gene in a biological sample derived from the patient with cancer,
wherein the fact that the measured expression level of the protein or gene is higher than that of a control indicates that a prognosis of the patient with cancer is not favorable.

10. The method according to Claim 9, wherein the cancer is bladder cancer, pancreatic cancer, or hepatocellular carcinoma.

11. A method for screening an anti-cancer agent, the method comprising: a step of measuring an expression level of an SPSB2 protein or an SPSB2 gene in a cancer cell cultured in the presence of a test substance,
wherein the fact that the expression level is significantly decreased compared with an expression level of the SPSB2 protein or the SPSB2 gene in absence of the test substance indicates that the test substance is an anti-cancer agent.

12. The method according to Claim 11, wherein the cancer cell is a cancer cell derived from bladder cancer, pancreatic cancer, or hepatocellular carcinoma.
